# EUROPEAN PATENT APPLICATION

(11) **EP 2 499 985 A1**
(43) Date of publication of application: **19.09.2012**
(21) Application number: 11157999.1
(22) Date of filing: 14.03.2011
(51) Int. Cl.: A61B 18/20, A61B 18/18, A61N 5/06

(54) **Light based skin care device with controllable fluency level**

(71) Applicant: Koninklijke Philips Electronics N.V., 5621 BA Eindhoven (NL)
(72) Inventor: Evers-Derkx, Lenie, J.,T., 5600 AE, Eindhoven (NL); Nuijs, Antonius, M., 5600 AE, Eindhoven (NL); Mikula, Christian, 5600 AE, Eindhoven (NL); Commissaris, Franciscus, A., C., M., 5600 AE, Eindhoven (NL)
(74) Representative: Damen, Daniel Martijn

(57) **Abstract**

A light based skin care device (10) is provided comprising a light source (11), user interface means (12), a control unit (13) and a skin sensor (14). The light source (11) is provided for providing light doses with a certain fluency level. The user interface (12) enables a user to set the fluency level to a value between a lower limit and an upper limit. The control unit (13) is coupled to the light source (11) and the user interface means (12) for controlling the light source (11) to provide the light doses with the light intensity set by the user. The skin sensor (14) can measure a value of a physical parameter of the user's skin and is coupled to the control unit (13) for setting the lower limit and the upper limit in dependence of the measured value of the physical parameter.

## Description

### FIELD OF THE INVENTION

This invention relates to a light based skin care device comprising a light source for providing light pulses with a fluency level, user interface means for enabling a user to set the fluency level to a value between a lower limit and an upper limit and a control unit, coupled to the light source and the user interface means for controlling the light source to provide the light pulses with the light intensity set by the user.

This invention further relates to a method and a computer program product for setting a fluency level for light pulses of a light source in a light based skin care device.

### BACKGROUND OF THE INVENTION

A broad range of skin care devices is on the market nowadays. Best known are the laser or Intense Pulsed Light (IPL) based epilators. Such devices use high intensity light pulses for removing hair from the body. Laser devices emit light of a single wavelength or in a narrow spectrum of light. IPL use xenon flash lamps that emit full spectrum light. The idea behind light based epilators is that the light pulses travel through the skin until it hits the melanin pigments that give a hair its color. The melanin absorbs the light energy and converts it to heat. The intense heat causes the hair to fall off or even destroys the hair together with its root and possibly the complete hair follicle.

IPL technology may also be used for other types of skin treatment. For example, IPL may be useful for removing wrinkles, treating pigmented lesions, sun damage induced dyspigmentation and vascular changes or for treating skin conditions like Poikiloderma of Civatte or Acne Rosacea.

Light based hair removal works best when the hairs to be removed comprise a lot of melanin and the treated skin does not. This is the case for lighter skinned people having dark hairs. For such people the fluency level of the applied light can be set to a relatively high value. The high intensity light pulses effectively remove the hairs, while the nearby skin tissue is hardly affected. People with darker skin colors, having more melanin in their skin, must use lower fluency levels to protect their skin from burning.

Current hair removal devices offer a number of fixed fluency level settings from which the user may choose. For example, settings between 2.0 and 6.8 J/cm² with 1.2 J/cm2 between each setting are available for selection. In the device manual, a table is provided showing for each skin type two or three recommended settings. For people with light skin, the highest fluency levels are advised, for people with darker skin the table advises to use the lower fluency levels. For people with black skin, the table even advises not to use the device at all. In some consumer hair removal devices, a melanin sensor is included for blocking use by users with too dark skin. In international patent application WO 2005/074830, a hair removal apparatus is disclosed comprising means for varying pulse parameters in order to reduce damage of the user's skin. A temperature measurement is included for monitoring a thermal reaction of the skin to an applied pulse. The thermal reaction may be used for automatically varying one or more pulse parameters.

From market surveys it is known that many users do not select the highest advised setting because of discomfort. Instead of using the highest advised fluency level, they use a lower setting which is at least 1.2 J/cm² lower. This leads to less effective hair removal and lower customer satisfaction.

### OBJECT OF THE INVENTION

It is an object of the invention to provide a light based skin care device with an increased chance that the user will select an effective fluency level.

### SUMMARY OF THE INVENTION

According to a first aspect of the invention, this object is achieved by providing a light based skin care device comprising a light source for providing light pulses with a fluency level, a user interface means, a control unit and a skin sensor. The user interface means are provided for enabling a user to set the fluency level to a value between a lower limit and an upper limit. The control unit is coupled to the light source and the user interface means for controlling the light source to provide the light pulses with the light intensity set by the user. The skin sensor is provided for measuring a value of a physical parameter of the user's skin, the skin sensor being coupled to the control unit for setting the lower limit and the upper limit in dependence of the measured value of the physical parameter.

The main advantage of the skin care device according to the invention is that, based on the measured skin parameter, a personalized range of available settings can be offered to the user. In the already known devices, low fluency level settings are offered to people with light skin. However, when these low fluency levels are selected, the device will be far less effective than possible. At the same time, high fluency levels are offered to people with dark skin, although such settings should not be used because of the expected damage to the skin. The device according to the invention blocks the less effective and possibly harmful options, without reducing the possibility to choose from different available settings. Moreover, the device according to the invention narrows the range between the lower limit and the upper limit and decreases the gap between two subsequent fluency level settings. A user can decide not to use the highest fluency level to avoid discomfort, without being forced to select a much too low fluency level.

The physical parameter may, e.g., be a color or a melanin content of the user's skin. The melanin content may be measured by determining a reflectance or absorption spectrum for light incident on the tested skin. The skin sensor may, e.g. measure a color of reflected light or local thermal effects of incident radiation. Other skin parameters that may be relevant for determining lower and upper limits of the fluency level may, e.g., be humidity or roughness.

The user interface means may offer a fixed number of available fluency levels in between the lower limit and the upper limit (wherein 'between' is considered to include the lower limit and the upper limit). Alternatively, a slider or control knob may be provided for offering the user a continuum fluency levels to choose from.

The light based skin care device according to the invention may be a laser based or IPL based hair removal device. Also other types of light pulse based skin care devices requiring different settings for different skin types may use the inventive concepts of the current invention. The invention may also be used in combination with continuous light sources. For example, a hair removal device employing a continuous light source is disclosed in the international patent application published as WO 2005/016453.

These and other aspects of the invention are apparent from and will be elucidated with reference to the embodiments described hereinafter.

### BRIEF DESCRIPTION OF THE DRAWINGS

In the drawings:
Figure 1 shows a hair removal device according to the invention,
Figure 2 shows a block diagram of a skin care device according to the invention, and
Figure 3 shows a flow chart of a method of setting a fluency level according to the invention.

### DETAILED DESCRIPTION OF THE INVENTION

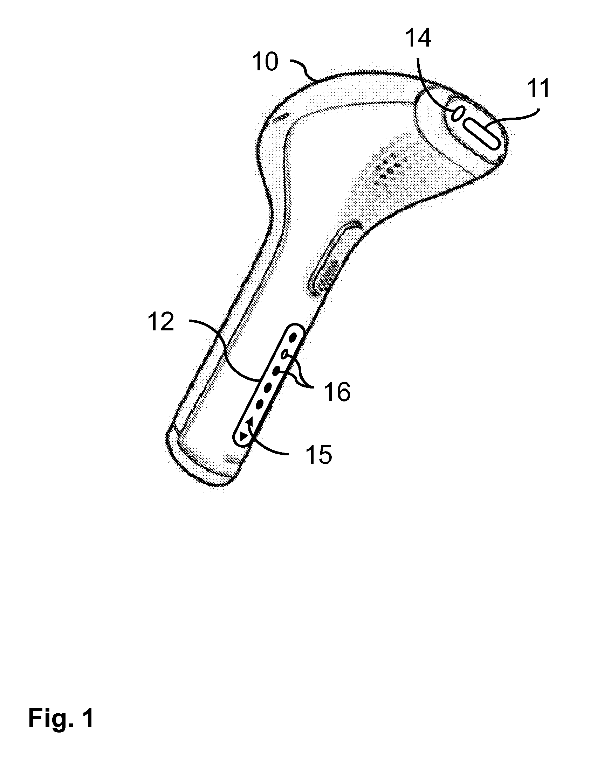

Figure 1 shows a hair removal device 10 according to the invention. The hair removal device 10 comprises a light source 11 for providing light pulses to the skin to be treated. The light source 11 may be a laser source emitting high energy light pulses at a single wavelength or in a narrow range of wavelengths. In most consumer hair removal devices 10 the light source 11 Intense Pulsed Light (IPL) is used. IPL produces light of high intensity during a very short period of time. For example, xenon flash lamps and focusing optics may be used to produce the required light pulses.

A skin sensor 14 is placed close to the light source 11. The skin sensor 14 is provided for measuring a value of a physical parameter of the user's skin. The measured physical parameter may, e.g., be a melanin content of the user's skin. The melanin content may be measured by determining a reflectance or absorption spectrum for light incident on the tested skin. The skin sensor 14 may, e.g. measure a color of reflected light or local thermal effects of incident radiation. Other skin parameters that may be relevant for determining lower and upper limits of the fluency level may, e.g., be humidity or roughness.

The measured skin parameter is used to determine the lower limit and upper limit of the fluency level of the light pulses to be applied. The upper limit should be such that the user is protected against light pulses with an intensity that would cause too much damage of the treated skin areas. The lower limit assures that the selected light intensity will be sufficiently high for effectively removing hair from the user's skin. More than one skin parameter, possibly measured by more than one skin sensor, may be used for setting the lower an upper limit.

In order to determine the lower limit and the upper limit, the measured skin parameter may be used for determining a skin type and a look-up table may give the appropriate limit for the determined skin type. For a user with skin type II (beige, easy sunburn, minimal tanning) the lower and upper limits may, e.g., be set at 5.6 and 6.8 J/cm², respectively. For a user with skin type V (dark brown, rarely sunburn, very good tanning) the lower and upper limits may, e.g., be set at 2.0 and 4.4 J/cm², respectively. Instead of using look-up tables, the skin parameter may also be calculated directly from the measured skin parameter.

A user control means 12 is provided to enable the user to set the fluency level to a desired level. In this example, the device 10 can be operated in five different settings. The five different settings are represented by five indicators 16. The lowest indicator 16 (further away from the light source 11) corresponds to the lower limit of the fluency level. The upper indicator 16 (closest to the light source 11) corresponds to the upper limit of the fluency level. If the lower limit and the upper limit are, e.g., determined to be 4.4 and 6.8 J/cm², the five settings may be 4.4, 5.0, 5.6, 6.2 and 6.8 J/cm².

In this figure, the fourth indicator 16 is on and the other indicators 16 are off, which means that the device is configured to provide light pulses with an intensity value of one step below the upper limit. For all users this setting will be one setting below the upper limit. However, for some users this setting may, e.g., correspond to a fluency level of 6.2 J/cm², while for another user this setting will correspond to 3.8 J/cm² or some other value.

The user control means 12 shown in this figure further comprise two buttons 15 for increasing (up arrow) or decreasing (down arrow) the fluency level. It is to be noted that the shown user control means 12 are just used as an example. Many alternative implementations of similar functionality are possible. For example, a slider or control knob may be used for offering the user a continuum of selectable intensity values between the lower and upper limit. The user control means 12 may include a small display for showing the currently selected setting and/or the available settings.

Placing the skin sensor 14 close to the light source 11 makes it possible to use the sensor 14 without requiring the user to perform additional acts. When the user wants to use the device 10 and places it against his skin, the device 10 can perform the skin parameter measurement before the light source 14 is used for removing hair. After the skin parameter measurement, the light source 14 starts providing light pulses with the selected intensity. The skin sensor 14 may even be integrated with the light source 11. Such integration would be particularly advantageous when the skin sensor 14 needs a light source for performing the skin parameter measurement. If the skin sensor 14 would be placed at a surface of the device 10 that, during use, is not in contact with the user's skin, the user would have to perform a separate act of measuring the skin parameter.

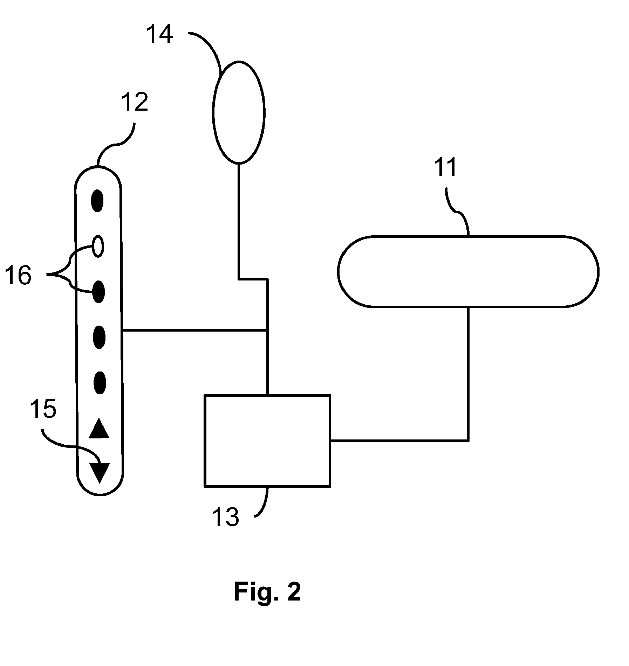

Figure 2 shows a block diagram of a skin care device 10 according to the invention. In addition to the features already described above, this figure also shows the control unit 13, to which the skin sensor 14, user control means 12 and the light source 11 are coupled. The control unit 13 controls the functioning of the different features. For example, it converts the measured skin parameter to a lower and upper limit. It determines the fluency levels that are available for selection and receives, from the user control means 12, a fluency level selected by the user. The selected fluency level is used for operating the light source 11 according to the user's preference.

It is to be noted that the skin sensor 14 is not necessarily integrated in the device 10 shown in figure 1. The skin sensor 14 may be a completely separate unit. The user then first uses the skin sensor 14 for determining the skin parameter or a skin type. The skin parameter is then communicated to the control unit. This communication may be realized through some data cable or wireless. Alternatively, the skin sensor 14 has some display means for informing the user about the measured skin parameter or a determined skin type and the user uses the user control means 12 for providing this skin parameter or skin type to the control unit 13. If the skin sensor 14 is a separate unit, the measured skin parameter, determined skin type or lower and upper limit may be stored in a memory of the device 10. If such parameters are stored in the device 10 it is not necessary to use the skin sensor 14 every time the device 10 is used.

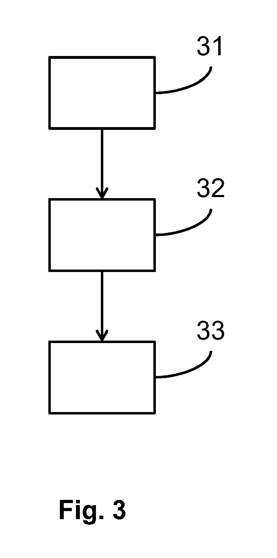

Figure 3 shows a flow chart of a method of setting a fluency level according to the invention. The method at least includes a measurement step 31, a limitation step 32 and a value setting step 33. During the measurement step 31 a value of a physical parameter of the user's skin is measured using the skin sensor 14. In limitation step 32, the control unit 13 defines the lower limit and the upper limit for the fluency level in dependence of the measured value of the physical parameter. After setting the lower and upper limit, the control unit 13 may also determine the fluency levels corresponding to available intermediate settings. In value setting step 33 the user selects a setting between the lower limit and the upper limit. The selected setting will be used when the device 10 is used for treating the user's skin.

It will be appreciated that the invention also extends to computer programs, particularly computer programs on or in a carrier, adapted for putting the invention into practice. The program may be in the form of source code, object code, a code intermediate source and object code such as partially compiled form, or in any other form suitable for use in the implementation of the method according to the invention. It will also be appreciated that such a program may have many different architectural designs. For example, a program code implementing the functionality of the method or system according to the invention may be subdivided into one or more subroutines. Many different ways to distribute the functionality among these subroutines will be apparent to the skilled person. The subroutines may be stored together in one executable file to form a self-contained program. Such an executable file may comprise computer executable instructions, for example processor instructions and/or interpreter instructions (e.g. Java interpreter instructions). Alternatively, one or more or all of the subroutines may be stored in at least one external library file and linked with a main program either statically or dynamically, e.g. at run-time. The main program contains at least one call to at least one of the subroutines. Also, the subroutines may comprise function calls to each other. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the means of at least one of the systems and/or products set forth. These instructions may be subdivided into subroutines and/or be stored in one or more files that may be linked statically or dynamically.

The carrier of a computer program may be any entity or device capable of carrying the program. For example, the carrier may include a storage medium, such as a ROM, for example a CD ROM or a semiconductor ROM, or a magnetic recording medium, for example a floppy disc or hard disk. Further the carrier may be a transmissible carrier such as an electrical or optical signal, which may be conveyed via electrical or optical cable or by radio or other means. When the program is embodied in such a signal, the carrier may be constituted by such cable or other device or means. Alternatively, the carrier may be an integrated circuit in which the program is embedded, the integrated circuit being adapted for performing, or for use in the performance of, the relevant method.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A light based skin care device (10) comprising:
- a light source (11) for providing light doses with a fluency level,
- user interface means (12) for enabling a user to set the fluency level to a value between a lower limit and an upper limit,
- a control unit (13), coupled to the light source (11) and the user interface means (12) for controlling the light source (11) to provide the light doses with the light intensity set by the user,
- a skin sensor (14) for measuring a value of a physical parameter of the user's skin, the skin sensor (14) being coupled to the control unit (13) for setting the lower limit and the upper limit in dependence of the measured value of the physical parameter.

2. A light based skin care device (10) as claimed in claim 1, wherein the skin sensor (14) is a melanin sensor and the physical parameter is a melanin content of the user's skin.

3. A light based skin care device (10) as claimed in claim 1, wherein the skin sensor (14) is a thermal sensor and the physical parameter is an energy absorbance value of the user's skin.

4. A light based skin care device (10) as claimed in claim 1, wherein the user interface means (12) are arranged for enabling a user to select the fluency level from a limited number of at least two available fluency levels between the lower limit and the upper limit.

5. A light based skin care device (10) as claimed in claim 1, wherein the user interface means (12) are arranged for enabling a user to select the fluency level from a continuum of available fluency levels between the lower limit and the upper limit.

6. A light based skin care device (10) as claimed in claim 1, wherein the light source (11) is an Intense Pulsed Light source or a laser source.

7. A light based skin care device (10) as claimed in claim 1, wherein the skin care device (10) is a hair removal device.

8. A method of setting a fluency level for light pulses of a light source in a light based skin care device, the method comprising
- measuring (31) a value of a physical parameter of a user's skin,
- in dependence of the measured value of the physical parameter, defining (32) a lower limit and an upper limit for the fluency level,
- setting (33) the fluency level to a user defined value between the lower limit and the upper limit.

9. A computer program product for setting a fluency level for light pulses of a light source in a light based skin care device, which program is operative to cause a processor to perform the method as claimed in claim 8.
